# EUROPÄISCHE PATENTSCHRIFT

(11) **EP 1 073 712 B1**
(45) Veröffentlichungstag und Bekanntmachung des Hinweises auf die Patenterteilung: **28.06.2006**
(21) Anmeldenummer: 99920820.0
(22) Anmeldetag: 29.04.1999
(51) Int. Cl.: C12N 1/04, A23K 3/03, A23L 1/03, A23K 1/00, C12R 1/25

(54) **TROCKENE MIKROORGANISMENKULTUREN UND VERFAHREN ZU DEREN HERSTELLUNG**
DRIED MICROORGANISM CULTURES AND METHOD FOR PRODUCING SAME
CULTURES DE MICRO-ORGANISMES SECHES ET LEUR PROCEDE DE PREPARATION

(30) Priorität: 30.04.1998 DE 19819475
(43) Veröffentlichungstag der Anmeldung: 07.02.2001
(73) Patentinhaber: BASF Aktiengesellschaft, 67056 Ludwigshafen (DE)
(72) Erfinder: RUNGE, Frank, D-67133 Maxdorf (DE); COOPER, Bryan, D-68199 Mannheim (DE); BRÖCKEL, Ulrich, D-67251 Freinsheim (DE); HEINZ, Robert, D-67067 Ludwigshafen (DE); HARZ, Hans-Peter, D-67373 Dudenhofen (DE); EIDELSBURGER, Ulrich, D-67258 Hessheim (DE); KÄSLER, Bruno, D-67071 Ludwigshafen (DE); KELLER, Thomas, D-67308 Lautersheim (DE)
(74) Vertreter: Kinzebach, Werner
(86) Internationale Anmeldenummer: PCT/EP1999/002925
(87) Internationale Veröffentlichungsnummer: WO 1999/057242

(56) Entgegenhaltungen:
- EP-A- 0 520 748
- EP-A- 0 818 529
- FR-A- 2 247 253
- GB-A- 1 073 030
- GB-A- 2 016 043
- US-A- 3 407 072
- US-A- 3 536 498
- US-A- 3 897 307
- DATABASE WPI Section Ch, Week 8745 Derwent Publications Ltd., London, GB; Class D13, AN 87-319054 XP002113266 & SU 1 292 706 A (APPL BIOCHEM RES), 28. Februar 1987 (1987-02-28)

## Beschreibung

Die vorliegende Erfindung betrifft neuartige trockene Mikroorganismenkulturen, welche insbesondere zur Herstellung von Nahrungs- und Futtermitteln brauchbar sind, sowie Verfahren zur Herstellung trockener Mikroorganismenkulturen.

Ein Hauptanwendungsgebiet von Mikroorganismen, wie Bakterien und Hefen, ist die Herstellung von Nahrungsmitteln und Futtermitteln. So werden beispielsweise Milchsäurebakterien, wie zum Beispiel solche der Gattung Streptococcus sp. oder Lactobacillus sp., bei der Herstellung von Milchprodukten, wie Sauerrahm, Buttermilch, Joghurt, Kefir, Kumis, Quark sowie bei der Herstellung von Sauerteig und zur Konservierung von Rohwurst, wie z. B. Salami, verwendet. Bei der Herstellung von Futtermitteln, wie z. B. Silage, kommen ebenfalls Milchsäurebakterien, wie z. B. solche der Gattung Lactobacillus sp., zum Einsatz.

Die zur Herstellung von Nahrungs- und Futtermitteln erforderlichen Mikroorganismenpräparate kommen gewöhnlich in Form sogenannter Starterkulturen zum Einsatz. Hierbei handelt es sich zumeist nicht um frisch hergestellte Flüssigkulturen, sondern entweder um gewöhnlich in flüssigem Stickstoff tiefgefrorene Kulturen oder Trockenpräparate. Trockenpräparate sind üblicherweise bevorzugt, da deren Transport und Lagerung im Vergleich zu tiefgefrorenen Präparaten technisch weniger aufwendig ist.
Aus dem Stand der Technik sind unterschiedlichste Formen trockener Zubereitungen von Mikroorganismenkulturen bekannt. So beschreibt beispielsweise die EP-A-0 131 114 ein Lactobacilluspräparat, wobei eine Zellsuspension der Bakterien auf eine pulver- oder granulatförmige Trägermasse aufgetragen und getrocknet wird. Zur Lagerung des Präparates ist es jedoch erforderlich, dieses in einer sauerstofffreien Schutzgasatmosphäre zu verpacken. In der DD 840493952 wird vorgeschlagen, kultivierte Mikroorganismenstämme zur Herstellung von Starterkulturen gefrierzutrocknen, in Folie zu verpacken und bei 279 bis 288 Kelvin zu lagern. Die JP-A-06/217713 beschreibt die Herstellung spezieller Lactobacillus-Starterkulturen durch Sprühtrocknung. In der EP-A-0 202 409 wird vorgeschlagen, Trockenkulturen einer Nassgranulierung zu unterziehen, das Granulat zu kugelförmigen Partikeln zu verarbeiten und anschließend zu trocknen. Außerdem wird in einer Reihe von Publikationen vorgeschlagen, beschichtete trockene Bakterienpräparate bereitzustellen (vgl. z. B. US-A-3,677,897).

Für die Herstellung trockener Mikroorganismenpräparate werden im Stand der Technik eine Reihe unterschiedlicher Verfahren beschrieben. Neben den oben bereits erwähnten Gefriertrocknungs- und Wirbelschichttrocknungsverfahren stellt eine weitere Herstellungsalternative die Sprühtrocknung einer Mikroorganismensuspension dar. So beschreiben beispielsweise Stadhouders, J. et al., in Neth. Milk Dairy J. (1969) 23, 182 die Sprühtrocknung von Milchsäurebakterien bei 70 °C, gekoppelt mit einem Nachtrocknungsschritt bei 27 °C im Vakuum. Zur Trocknung wird offensichtlich keine vorkonditionierte, d. h. vorgetrocknete Luft verwendet. Dem Sprühgut wird vor der Trocknung eine Calciumhydroxidaufschlämmung zugesetzt. Das bei der Sprühtrocknung gebildete Calciumlactat ist insofern von Vorteil, als es eine geringere Hygroskopizität aufweisen soll. In anderen, aus dem Stand der Technik bekannten Sprühtrocknungsverfahren werden Bakteriensuspensionen versprüht, denen zuvor verschiedenste Trägermaterialien zugesetzt wurden. So wird beispielsweise gemäß SU 724113 eine mit Trockenmilchpulver, Melasse und Natriumglutamat versetzte Bakteriensuspension versprüht. Gemäß SU 1616990 wird eine mit dem Mineral Palygorskit versetzte Bakteriensuspension sprühgetrocknet. Die WO-A-88/06181 beschreibt die Sprühtrocknung einer mit Ton versetzten Bakteriensuspension. Die JP-A-69/67989 beschreibt die Sprühtrocknung von Hefe- oder Bakterienzellen, welche in einer neutralen oder schwach sauren Lösung suspendiert sind, die Proteine, Carboxymethylcellulose, Alginat oder Alginatester, Di- oder höhere Saccharide oder mehrwertige Alkohole enthält. Die US 3,897,307 beschreibt ein Verfahren zur Herstellung einer trockenen, trägergebundenen Lactobacillus-Kultur unter Verwendung eines stabilisierenden Additivs.

Die bisher aus dem Stand der Technik bekannten trockenen Mikroorganismenpräparate, insbesondere solche, welche für die Herstellung von Nahrungs- oder Futtermitteln Verwendung finden, weisen wenigstens einen der folgenden Nachteile auf:
1) Der Gehalt lebensfähiger Keime pro Gewichtseinheit des Trokkenmaterials ist herstellungsbedingt sehr gering, so dass große Volumina des Trockenpräparates bei der Endanwendung einzusetzen sind;
2) die Lagerstabilität ist zu gering, so dass die Trockenpräparate innerhalb weniger Wochen verbraucht werden müssen, wenn keine Lagerung unter technisch aufwendigen Bedingungen möglich ist;
3) die Trockenpräparate weisen einen hohen Staubanteil auf, wodurch deren Verarbeitung erschwert wird;
4) die mechanische Stabilität ist sehr gering, so dass bei Abmischungen der Präparate mit mineralischen Zusätzen ein feinteiliger Abrieb gebildet wird und eine Entmischung des Feststoffpräparates zu beobachten ist;
5) die Lösungsgeschwindigkeit der Trockenpräparate ist nicht zufriedenstellend, so dass der gewünschte mikrobiologische Prozess für die Herstellung des Nahrungs- oder Futtermittels nur langsam anläuft und unerwünschten Mikroorganismen die Möglichkeit zur Vermehrung gegeben wird, was zu erheblichen Qualitätsverlusten führen kann.

Auch die aus dem Stand der Technik bisher bekannten Herstellungsverfahren, insbesondere die bisher beschriebenen Sprühtrocknungsverfahren, sind aus wenigstens einem der folgenden Gründe nicht zufriedenstellend:
1) die Verfahren sind technisch sehr aufwendig;
2) die Überlebensrate der Mikroorganismen bei der Trocknung ist zu gering;
3) der Feuchtegehalt des Trockenproduktes ist zu hoch.

Eine erste Aufgabe der vorliegenden Erfindung betrifft somit die Bereitstellung verbesserter trockener Mikroorganismenkulturen, welche die oben genannten, aus dem Stand der Technik bekannten Mängel weitgehend nicht mehr aufweisen. Insbesondere sollen gegenüber dem Stand der Technik verbesserte Starterkulturen bereitgestellt werden. Die erfindungsgemäßen Starterkulturen sollen vor allem eine verbesserte Herstellung von Silage ermöglichen.

Eine zweite Aufgabe der vorliegenden Erfindung besteht in der Bereitstellung verbesserter Verfahren zur Herstellung trockener Mikroorganismenkulturen. Insbesondere sollte ein verbessertes Verfahren zur Sprühtrocknung von Mikroorganismenkulturen bereitgestellt werden, welches die Herstellung von Trockenpräparaten mit einem hohen Gehalt an lebensfähigen Keimen und hoher Lagerstabilität ermöglicht.

Gelöst wird obige erste Aufgabe durch Bereitstellung einer trokkenen Mikroorganismenkultur, enthaltend wenigstens eine Mikroorganismen-Spezies in geträgerter Form, dadurch gekennzeichnet, dass die Kultur in Form von Partikeln vorliegt, welche
a) eine Partikelgröße von wenigstens etwa 0,1 mm aufweisen,
b) etwa 10¹⁰ bis 4·10¹¹ cfu/g wenigstens eines Mikroorganismen-Spezies enthalten; und
c) verdichtet sind.

Die erfindungsgemäßen partikelförmigen Kulturen sind aufgrund der gewählten Korngröße praktisch staubfrei. Der Staubanteil liegt vorzugsweise im Bereich von etwa 0,01 bis 0,05 Gew.-%, bezogen auf das Gesamtgewicht der Trockenkultur. Dies entspricht einer mit einem Casella-Gerät gravimetrisch bestimmten Staubzahl im Bereich von etwa 1 bis 12 .

Die erfindungsgemäßen Partikel besitzen außerdem einen verdichteten, d.h. kompakten Aufbau. Diesen errreicht man vorzugsweise durch einen später genauer erläuterten und bisher für trockene Mikroorganismenpräparate bisher nicht beschriebenen Verdichtungsschritt bei deren Herstellung. Dabei wird ein, beispielsweise durch Sprüh-, Gefrier- oder Wirbelschichttrocknung erhaltenes pulverförmiges Vorprodukt (wie z.B. das später beschriebene, mit einer erfindungsgemäßen Sprühtrocknungsvariante erhältliche Pulverkonzentrat), das gewöhnlich einen signifikanten Staubanteil (z.B. eine Staubzahl von etwa 25 bis 100) aufweist, mechanisch verdichtet.

Die Verdichtung kann beispielsweise dadurch erfolgen, daß man das pulverförmige Vorprodukt unter Einwirkung von Linienkräften, z.B. im Bereich von etwa 5 bis etwa 25 kN/cm, insbesondere etwa 10 bis etwa 15 kN/cm, z. B. in herkömmlichen Kompaktiervorrichtungen kompaktiert. Das Vorprodukt kann aber auch unter Einwirkung von Drücken im Bereich von etwa 50 bis etwa 250 MPa, insbesondere im Bereich von etwa 80 bis 200 MPa, wie z.B. etwa 90 bis etwa 160 MPa, z.B. in üblichen Tablettenpressen, tablettiert werden. Besonders bevorzugt ist die Verdichtung durch Kompaktieren. Weiterhin bevorzugt ist die Kompaktierung erfindungsgemäß durch Sprühtrocknen erhaltener Pulverkonzentrate.

Durch die Bereitstellung von Mikroorganismenkulturen des oben beschriebenen Typs wird überraschenderweise erreicht, dass die Verarbeitung, insbesondere als Starterkulturen, deutlich vereinfacht wird und außerdem die mechanische Stabilität der Partikel und somit die Gefahr der Entmischung von Starterkulturpräparaten deutlich verringert wird. Überraschenderweise wurde auch festgestellt, daß die Verdichtung des pulverförmigen Vorprodukts die Produktqualität bezüglich der Anzahl lebensfähiger Keime praktisch nicht beeinträchtigt. Vielmehr wird durch die erzielte hohe Dichte das Eindringen von Luft und Feuchtigkeit in die erfindungsgemäßen Trockenpräparate so signifikant verringert, daß eine wesentliche Verbesserung der Lagerstabilität erreicht werden kann. So konnten beispielsweise Überlebensraten von 60% und mehr nach einjähriger Lagerung bei Raumtemperatur erzielt werden. Derart vorteilhafte Lagerstabilitätsdaten wurden bisher nicht beschrieben.

Insbesondere können die verdichteten Partikel kompaktiertes Brechgut (d.h. durch Zerkleinern und gegebenenfalls Klassieren von kompaktierten Produktsträngen erhaltenes Material) mit einem Durchmesser von etwa 0,1 mm bis etwa 2 mm, vorzugsweise 0,3 bis 1,25 mm, umfassen. Der Durchmesser stellt dabei einen rechnerisch aus der Massensummenverteilung der verdichteten Partikel ermittelten Wert dar und entspricht dem Durchmesser massengleicher Kugeln. Die Bruchkantenlänge der Partikel liegt etwa im Bereich von 0,1 bis 2 mm, insbesondere etwa 0,1 bis 1,4 mm.

Die verdichteten Partikel können außerdem als Tabletten beliebiger Form, wie z.B. rund, eckig oder oval, mit einem Durchmesser von etwa 2 bis 50 mm und einem Verhältnis von Durchmesser zu Dicke von etwa 1:0,1 bis etwa 10:1, insbesondere etwa 1:1 bis etwa 5:1, vorliegen.

Gemäß einer weiteren bevorzugten Ausführungsform der Erfindung enthalten die trockenen Mikroorganismenkulturen als weitere Komponente einen Brausezusatz, umfassend eine Säurekomponente, wie z. B. eine organische, nichtflüchtige Carbonsäure, und eine gasbildende Komponente, wie z. B. eine CO₂-bildende Komponente. Derartige Brauseformulierungen besitzen den besonderen Vorteil einer überraschend schnellen Auflösung nach Applikation der Starterkultur. Als Folge dieser schnellen Auflösung der Starterkultur in dem sie umgebenden Milieu ist eine rasche Vermehrung der Starterkulturmikroorganismen gewährleistet, wodurch Qualitätsverluste des mit Hilfe der Starterkultur herzustellenden Produkts überraschend gut vermeidbar sind.

Vorzugsweise enthält eine erfindungsgemäße verdichtete Trockenkultur als Träger wenigstens ein Matrixmaterial zur Einbettung der Mikroorganismenzellen und gegebenenfalls wenigstens ein weiteres, die Zellen stabilisierendes Additiv.

Der in den erfindungsgemäßen Trockenkulturen verwendete Träger umfasst wenigstens eine, üblicherweise frisch gezüchteten Mikroorganismen vor der Trocknung als Coformulans zugesetzte Matrixkomponente, ausgewählt unter Mono-, Oligo- und Polysacchariden, Polyolen, Polyethern, Polymeren, wie CMC oder PVP, Oligo- und Polypeptiden, aus natürlichen Quellen, wie z.B. Milch, Fleisch oder Getreide, abgeleitete Stoffe oder Stoffgemische, wie z.B. Süßmolkepulver, Weizengrießkleie, Pepton, Alginate, mineralischen Verbindungen, oder Gemischen solcher Matrixsubstanzen. Weiterhin können stabilisierend wirkende Additive zusammen mit der Matrixsubstanz oder später zugesetzt werden, beispielsweise Antioxidanzien, wie α-Tocopherol oder Ascorbinsäure, oder Gemische davon. Darüber hinaus können weitere Substanzen stabilisierend wirken, die ausgewählt sind unter anorganischen Salzen, wie Alkali- oder Erdalkalichloride, anorganischen oder organischen Puffern, wie Alkaliphosphatpuffer, Aminosäuren, wie Asparagin- oder Glutaminsäure und den Salze davon, organischen Carbonsäuren, wie Citronensäure, organischen, nichtflüchtigen Lösungsmitteln, wie DMSO, und weiteren Verbindungen, wie β-Carotin und Gemischen solcher Additive.

Die erfindungsgemäßen Mikroorganismenkulturen umfassen vorzugsweise lebensfähige Mikroorganismen in einer Konzentration von 10¹⁰ bis 10¹² cfu (colony forming units)/g Trockenkultur. Die erfindungsgemäß hergestellten Pulverkonzentrate enthalten etwa 4·10¹¹ bis 1·10¹², vorzugsweise etwa 4·10¹¹ bis 8·10¹¹ cfu/g. Die erfindungsgemäßen verdichteten Kulturen enthalten etwa 1·10¹¹ bis 4·10¹¹, insbesondere etwa etwa 3·10¹¹ cfu/g . Starterkulturen zur Silageherstellung enthalten etwa 1 bis 7·10¹⁰, insbesondere etwa 3·10¹⁰ cfu/g.

Dabei können die Mikroorganismen von einer oder mehreren Mikroorganismenspezies abgeleitet sein. Eine besonders bevorzugte Spezies sind Milchsäure produzierende Bakterien, wie z.B. solche, die zur Silageherstellung geeignet sind, wie beispielsweise Lactobacillus plantarum.

Silage im Sinne der vorliegenden Erfindung umfaßt durch Einwirkung von Mikroorganismen haltbar gemachten Futterpflanzenprodukte, beispielsweise basierend auf Gras, Klee, Stroh, Maispflanzen, Futterrüben, Hülsenfrüchten, Getreide, wie z.B. Mais und Weizen, und dergleichen.

Die oben beschriebene zweite Aufgabe der vorliegenden Erfindung wird überraschenderweise gelöst durch Bereitstellung eines Sprühtrocknungsverfahrens zur Herstellung einer trockenen Mikroorganismenkultur, enthaltend wenigstens eine Mikroorganismen-Spezies in geträgerter Form, dadurch gekennzeichnet, dass man
a) in einer wenigstens eine Mikroorganismen-Spezies enthaltende Flüssigkeit wenigstens eine zur Ausbildung eines Trägers geeignete Substanz löst oder suspendiert,
b) das so erhaltene Gemisch in einem Sprühtrockner trocknet, wobei man zur Sprühtrocknung ein konditioniertes, getrocknetes und auf eine Temperatur im Bereich von mehr als etwa 80 °C, insbesondere auf etwa 90 bis etwa 135 °C, vorzugsweise etwa 100 bis etwa 110 °C, wie z.B. etwa 105 °C erhitztes Gas mit einem Taupunkt von etwa -10 bis -50°C verwendet, und
c) das Trockengut aus dem Sprühtrockner entfernt, wobei dieses eine Austrittstemperatur von etwa 45 bis 75 °C, vorzugsweise etwa 50 bis 65 °C, wie z.B. etwa 55 °C, aufweist.

Dieses erfindungsgemäße Sprühtrocknungsverfahren wird im folgenden auch als trägergebundenes Sprühtrocknungsverfahren bezeichnet. Das zur Trocknung verwendete Gas ist ein getrocknetes Gas mit einem Taupunkt von etwa -10 bis etwa -50 °C, wie z. B. konditionierte Pressluft oder konditionierter Stickstoff. Beispielsweise kann Pressluft mit einem Taupunkt von etwa -25 °C und Stickstoff mit einem Taupunkt von etwa -40 °C eingesetzt werden. Ein Taupunkt von +5 °C entspricht in etwa 5 g Wasser pro m³ Luft.

Gemäß einer bevorzugten Ausführungsform des erfindungsgemäßen Sprühtrocknungsverfahrens wird in einer nachgeschalteten, weiteren Stufe d) das Trockengut einer Nachtrocknung unterzogen. Die Nachtrocknungstemperatur liegt im Bereich von etwa 15 bis 50 °C, wie z. B. bei etwa 25 bis 40 °C. Die Nachtrocknung erfolgt beispielsweise in einer Gasatmosphäre oder im Vakuum; alternativ dazu besteht außerdem die Möglichkeit, ein Trocknungsmittel mit dem gemäß Stufe c) erhaltenen trockenen Mikroorganismenpräparat homogen zu vermischen.

Aufgrund seiner Auslegung gestattet das erfindungsgemäße Sprühtrocknungsverfahren überraschenderweise die Trocknung von Mikroorganismensuspensionen mit Überlebensraten bis zu 100 %. Aufgrund der Verwendung von konditioniertem Gas bei der Sprühtrocknung sowie dem optionalen Nachtrocknungsschritt werden überraschenderweise Trockenpräparate mit extrem niedrigem Feuchtegehalt (von etwa 2 bis 3 Gew.-% Wasser), entsprechend einer Wasseraktivität a_{w} von 0,03 bis 0,15, zur Verfügung gestellt. Dies hat unmittelbar zur Folge, dass die erfindungsgemäß sprühgetrockneten und gegebenenfalls nachgetrockneten Mikroorganismenkulturen Überlebensraten von bis zu 60 % nach einjähriger Lagerung bei Umgebungstemperatur und Umgebungsluft aufweisen.

Aufgrund der Überraschend hohen Überlebensrate bei der oben beschriebenen Sprühtrocknung ist der Gehalt an lebensfähigen Mikroorganismen ausgeprägt hoch. Das erhaltene Sprühtrocknungsprodukt wird daher auch als Pulverkonzentrat bezeichnet und kann zur Verringerung der Konzentration lebensfähiger Zellen je nach Anwendungsgebiet weiter verdünnt werden. Das Pulverkonzentrat eignet sich besonders zur Herstellung der oben beschriebenen erfindungsgemäßen verdichteten, partikelförmigen Kulturen.

Gegenstand der vorliegenden Erfindung ist daher außerdem ein Verfahren zur Herstellung der oben beschriebenen, verdichteten Mikroorganismenkulturen, wobei man
i) eine flüssige Mikroorganismenkultur durch trägergebundene Sprühtrocknung in ein Pulverkonzentrat der Mikroorganismenkultur überführt, welches 4·10¹¹ bis 1·10¹² cfu/g der Mikroorganismen enthält und das Trockengut eine Wasseraktivität von weniger als 0,4 aufweist, und
ii) das Pulverkonzentrat gegebenenfalls mit einem oder mehreren Coformulantien versetzt und
iii) diese Mischung durch Kompaktieren oder Tablettieren verdichtet.

Vorzugsweise wird die verdichtete Mischung in einem weiteren Verfahrensschritt gebrochen, d.h. zerkleinert, und gegebenenfalls unter Verwendung eines Siebs mit geeigneter Maschenweite zu verdichtetem Granulat der gewünschten Größe klassiert.

Unter "trägergebunden" versteht man dabei die Anwesenheit zumindest eines Matrixmaterials des oben beschriebenen Typs bei der Trocknung.

Gemäß einer bevorzugten Ausführungsform der oben genannten Kompaktierungs- oder Tablettierungs- bzw. Agglomerierungsverfahren wird Stufe i) insbesondere entsprechend dem oben beschriebenen Sprühtrocknungsverfahren durchgeführt.

Das durch die oben beschriebenen Verdichtungsverfahren erhaltene Produkt wird im Rahmen der vorliegenden Erfindung auch als verdichtetes oder kompaktiertes Trockenkonzentrat bezeichnet (cfu-Bereich etwa 1·10¹⁰ bis 1·10¹¹⁾ und kann als solches, z.B. als konzentrierte "Starterkultur, vertrieben werden.

Ein weiterer Gegenstand der vorliegenden Erfindung betrifft die Verwendung der erfindungsgemäßen verdichteten, trockenen Mikroorganismenkulturen als Starterkulturen für die Herstellung von Nahrungsmitteln, wie z. B. für die Herstellung von Milchprodukten, wie Sauerrahm, Buttermilch, Joghurt, Kefir, Kumis, Quark, für die Herstellung von Sauerteig, Rohwurst, sowie für die Herstellung von Futtermitteln, wie z. B. Silage. Zu diesem Zweck wird die Kultur gegebenenfalls nach Auflösen mit dem Nahrungs- oder Futtermittelsubstrat vermischt. Sollte die Zellzahl in der Starterkultur zu hoch sein, kann eine Verdünnnung z.B. durch Vermischen mit einem inerten Feststoff, wie z.B. Kalk, insbesondere Futterkalk, angebracht sein.

Gegenstand der vorliegenden Erfindung sind außerdem Nahrungs- und Futtermittel, welche mit Hilfe der erfindungsgemäßen Starterkulturen hergestellt worden sind.

Die vorliegende Erfindung wird in den nun folgenden Abschnitten unter Bezugnahme auf die beiliegende Figur genauer beschrieben.

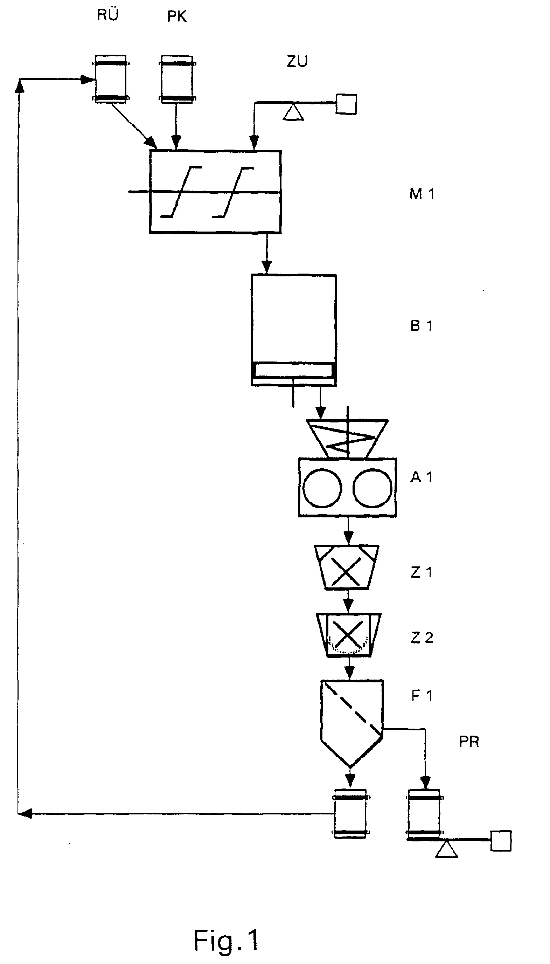

Figur 1 zeigt schematisch eine mögliche Herstellungweise von erfindungsgemäß kompaktiertem Granulat aus Pulverkonzentrat.

### Verwendbare Mikroorganismen

Die vorliegende Erfindung ist grundsätzlich nicht auf bestimmte Mikroorganismenkulturen beschränkt. Vielmehr erkennt der Fachmann, dass die vorliegende Erfindung auf jegliche Mikroorganismen, insbesondere Bakterien und Hefen, anwendbar ist, welche unter den in vorliegender Beschreibung angegebenen Bedingungen in ein trockenes Mikroorganismenpräparat überführbar sind. Eine geeignete Gruppe von Mikroorganismen, die erfindungsgemäß anwendbar sind, stellt die Gruppe der Milchsäure-produzierenden Bakterien dar. Insbesondere handelt es sich hierbei um Bakterien, welche zur homofermentativen Milchsäuregärung geeignet sind, d. h. Glucose über den Fructosebisphosphat-Weg zu Lactat abbauen. Typische Vertreter dieser Gruppe sind Bakterien der Gattungen Lactobacillus sp., Streptococcus sp. sowie Pediococcus sp. Als konkrete Beispiele für Lactobacillen können genannt werden Lactobacillus bulgaricus, Lactobacillus acidophilus, Lactobacillus helveticus, Lactobacillus bifidus, Lactobacillus casei, Lactobacillus lactis, Lactobacillus delbrueckii, Lactobacillus thermophilus, Lactobacillus fermentum, Lactobacillus brevis und Lactobacillus plantarum. Beispiele für geeignete Streptokokken sind Streptococcus lactis, Streptococcus cremoris, Streptococcus diacetilactis, Streptococcus thermophilus, Streptococcus pyrogenes, Streptococcus salivarius, Streptococcus faecalis, Streptococcus faecium; und Beispiele für geeignete Pediococcen sind Pediococcus cerevisiae und Pediococcus acidilactici.

### Fermentation der Mikroorganismen

Zur Durchführung der vorliegenden Erfindung verwendet man vorzugsweise frisch hergestellte Mikroorganismensuspensionen. Die für den jeweiligen Mikroorganismus optimalen Fermentationsmedien bzw. Fermentationsbedingungen sind entweder aus dem Stand der Technik bekannt oder können von dem mit der Aufzucht von Mikroorganismen betrauten Fachmann im Rahmen weniger Routineuntersuchungen bestimmt werden.

Gewöhnlich wird jedoch die Fermentation so durchgeführt, dass man, ausgehend von einer flüssigen oder halbfesten Vorkultur (Kulturvolumen etwa 10 bis 200 ml), frisch hergestelltes, steriles Fermentationsmedium unter sterilen Bedingungen animpft, wobei das Volumenverhältnis von Vorkultur zu Kulturmedium etwa 1:50 bis 1:200 betragen kann. Vorzugsweise verwendet man frisch gezüchtete Vorkulturen, welche sich in einer späten Phase des logarithmischen Wachstums befinden. Die Aufzucht erfolgt je nach zu züchtendem Mikroorganismus unter speziellen, optimierten Wachstumsbedingungen (wie Temperatur und pH). Gewöhnlich liegt die Aufzuchtstemperatur im Bereich von etwa 20 bis 40 °C, wobei jedoch beispielsweise bei Aufzucht thermophiler Bakterien deutlich höhere Temperaturen vorliegen können. Der Fermentationsansatz wird in gleichmäßiger Bewegung gehalten, beispielsweise durch moderates Rühren oder Einleiten von Luft oder Stickstoff, um die Ausbildung von Temperatur- oder Stoffgradienten zu verhindern und um auf diese Weise ein kontinuierliches Wachstum zu gewährleisten. Nach Beendigung der Wachstumsphase (beispielsweise bestimmt über das Erreichen einer bestimmten Zelldichte oder den Verbrauch eines der zugesetzten Nährstoffe), kann die Zellsuspension direkt zur Herstellung der erfindungsgemäßen Trockenpräparate eingesetzt werden.

Es besteht jedoch außerdem die Möglichkeit, die erhaltene ursprüngliche Zellsuspension zur Erhöhung der Zellzahl weiter aufzukonzentrieren. Geeignete Methoden hierfür sind beispielsweise Zentrifugation, Ultrafiltration oder Dünnschichtverdampfung. Üblicherweise verwendet man jedoch zur Aufkonzentrierung der Zellsuspension eines Zentrifugationsschritt, welcher vorzugsweise bei verringerter Temperatur, nämlich im Bereich von etwa 4 bis 10°C, durchgeführt wird.

Anstelle der Aufkonzentrierung oder in Kombination mit dieser besteht außerdem die Möglichkeit, die frisch gezüchtete Zellsuspension einem Waschschritt zu unterziehen, um die Aktivität möglicherweise negativ beeinflussende Kulturbestandteile, wie z. B. Stoffwechselprodukte, zu entfernen. Dabei geht man üblicherweise so vor, dass man, vorzugsweise bei etwa 4 bis 10°C, zunächst die ursprüngliche Kulturbrühe zu einer Suspension hoher Zelldichte aufkonzentriert und diese anschließend in einer geeigneten Pufferlösung aufnimmt und auf die gewünschte Zelldichte verdünnt. Erforderlichenfalls kann der Waschschritt auch mehrmals wiederholt werden. Erfindungsgemäß brauchbare Feststoffgehalte von Mikroorganismenkulturen, welche zur Herstellung der erfindungsgemäßen Trockenpräparate geeignet sind, liegen üblicherweise im Bereich von etwa 5 bis 25 Gew.-%, wie z.B. etwa 10 bis 20 Gew.-%.

Die Aufzucht der Mikroorganismen kann durch Batch-Fermentation oder kontinuierlich erfolgen.

Zur weiteren Veranschaulichung der Erfindung wird im folgenden Abschnitt die Aufzucht eines Milchsäure-bildenden Bakteriums, insbesondere Lactobacillus plantarum, näher beschrieben. Hierbei handelt es sich um ein insbesondere auf intakten und sich zersetzenden Pflanzen zu findendes Bakterium, welches besonders zur Herstellung von Silage-Futtermitteln geeignet ist.

Ein geeignetes Fermentationsmedium enthält pro Liter Medium etwa 40 bis 60 g Glucose, etwa 30 bis 60 g Hefeextrakt sowie einen Cocktail üblicher Spurenelemente, wie z. B. Magnesium, Mangan und gegebenenfalls Eisen. Der pH-Wert des Fermentationsmediums liegt bei etwa 6 bis 7. Die Fermentationstemperatur liegt bei etwa 33 bis 38°C. Durch Zugabe von steriler Natronlauge kann der pH-Wert des Fermentationsmediums im gewünschten Bereich gehalten werden.

Das Wachstum ist beendet, sobald kein Glucoseverbrauch bzw. keine Milchsäureneubildung mehr zu beobachten ist.

Gemäß einer erfindungsgemäß brauchbaren Variante der Lactobacillus-Fermentation wird nach Erreichen von etwa 80%igem oder 90%igem Wachstum die Temperatur des Fermentationsmediums auf etwa 42 bis 46 °C erhöht, bis die zugesetzte Glucose vollständig verbraucht ist. Erfindungsgemäß wurde festgestellt, daß sich auf diese Weise hergestellte Kulturen insbesondere bei der Sprühtrocknung besonders stabil verhalten, wodurch hohe Überlebensraten erzielbar sind. Vergleichbare Aufzuchtvarianten sind auch bei anderen erfindungsgemäß brauchbaren Mikroorganismen denkbar.

Nach beendetem Wachstum wird der Fermentationsansatz auf die gewünschte Zelldichte gebracht. Gewünschtenfalls kann die Zellsuspension gewaschen werden, bis sie praktisch lactatfrei ist. Die Zellzahl einer erfindungsgemäß brauchbaren Mikroorganismensuspension liegt gewöhnlich im Bereich von etwa 1 x 10¹⁰ bis etwa 5 x 10¹² cfu/g Suspension.

### Trägersubstanzen

Die erfindungsgemäß hergestellten trockenen Mikroorganismenkulturen enthalten neben gegebenenfalls nicht-flüchtigen Bestandteilen aus dem jeweiligen Fermentationsansatz, wie z. B. Stoffwechselprodukte, wenigstens ein Matrixmaterial und gegebenenfalls weitere stabilisierende Substanzen. Diese Coformulantien sind vorzugsweise ausgewählt unter anorganischen Salzen oder Puffern, wenigstens einer weiteren Verbindung, die ausgewählt ist unter Mono-, Oligo- und Polysacchariden, Polyolen, Polyethern, Aminosäuren, Oligo- und Polypeptiden, von Milch abgeleiteten Verbindungen, organischen Carbonsäuren, mineralischen Verbindungen, organischen Trägermaterialien, wie z. B. Weizengrießkleie, Alginaten, DMSO, PVP (Polyvinylpyrrolidon), CMC (Carboxymethylcellulose), α-Tocopherol, β-Carotin und Gemischen davon.

Als Beispiele für geeignete Saccharid-Trägerkomponenten sind zu nennen Saccharose, Fructose, Maltose, Dextrose, Lactose und Maltodextrin. Als Beispiel für ein geeignetes Polyol ist Glycerin zu nennen. Beispiele für geeignete Aminosäuren sind Glutaminsäure, Asparaginsäure und die Salze davon. Als Beispiel für einen geeigneten peptidischen Träger ist zu nennen Pepton. Als Beispiele für von Milch abgeleiteten Verbindungen ist neben oben genanntem Maltodextrin auch Süßmolkepulver zu nennen. Geeignete organische Carbonsäuren sind beispielsweise Zitronensäure, Äpfelsäure und 1-Ascorbinsäure. Beispiele für geeignete mineralische Träger sind Montmorillonit sowie Palygorskit.

Vorzugsweise verwendet man jedoch als Träger für die erfindungsgemäßen trockenen Mikroorganismenpräparate Gemische oben genannter Stoffklassen. Derartige Gemische umfassen vorzugsweise als Hauptkomponente ein Matrixmaterial, wie z. B. eine der oben genannten Saccharidkomponenten oder beispielsweise Süßmolkepulver, und gegebenenfalls einen Nebenanteil wenigstens einer weiteren Komponente, wie z. B. eine Pufferkomponente (beispielsweise Citronensäure) oder ein Antioxidans (beispielsweise 1-Ascorbinsäure oder α-Tocopherol). Der Zusatz weiterer stabilisierender Bestandteile, wie z. B. Natriumglutamat und/oder Pepton, hat sich ebenfalls als vorteilhaft erwiesen.

Die Matrixkomponente wird in erfindungsgemäß brauchbaren Trägergemischen üblicherweise in etwa 5- bis 30facher Menge der übrigen Trägerbestandteile verwendet. Beispiele für besonders geeignete Trägerkombinationen sind:
a) Süßmolkepulver/Citronensäure/1-Ascorbinsäure (Gewichtsverhältnis etwa 40:1:1).
b) Maltodextrin/Lactose/Citronensäure/1-Ascorbinsäure (Gewichtsverhältnis etwa 20:20:1:1), gegebenenfalls ergänzt mit etwa 1,5 Teilen β-Carotin und 0,5 Teilen α-Tocopherol je Teil Citronensäure.
c) Maltodextrin/Natriumglutamat/1-Ascorbinsäure (Gewichtsverhältnis etwa 10:1,5:1).
d) Lactose/Glucose/Pepton/Citronensäure (Gewichtsverhältnis etwa 6:6:1,2:1).

Die erfindungsgemäßen Trägersubstanzen können der Mikroorganismensuspension entweder als Feststoff oder in gelöster Form zugegeben werden. Vorzugsweise stellt man jedoch eine sterile Lösung des/der Träger her, kühlt diese auf eine Temperatur von 4 bis 10°C ab und vermischt diese mit der ebenfalls gekühlten Mikroorganismensuspension unter leichtem Rühren. Zur Herstellung einer homogenen Suspension rührt man das so erhaltene Gemisch unter weiterem Kühlen über einen Zeitraum von etwa 10 Minuten bis 1 Stunde.

### Herstellung trockener Mikroorganismenpräparate

Die in oben beschriebener weise mit dem Träger versetzte Mikroorganismensuspension kann nun in unterschiedlicher Weise getrocknet werden. Als Trocknungsverfahren eignen sich prinzipiell die Gefriertrocknung, die Wirbelschichttrocknung sowie, vorzugsweise die Sprühtrocknung. Unter Sprühtrocknung im Rahmen der vorliegenden Erfindung versteht man auch modifizierte Sprühtrocknungsverfahren, wie z. B. die Sprühagglomeration oder die agglomerierende Sprühtrocknung. Letzteres Verfahren ist auch unter der Bezeichnung FSD (Fluidized Spray Dryer) Verfahren bekannt.

Die Gefriertrocknung zur Herstellung erfindungsgemäßer trockener Mikroorganismenkulturen kann beispielsweise in Anlehnung an die in der EP-A-0 259 739 oder der US-A-3 897 307 beschriebenen Gefriertrocknungsverfahren durchgeführt werden. Auf den Inhalt dieser Druckschriften wird hiermit in vollem Umfang Bezug genommen.

Ein geeignetes Wirbelschicht-Trocknungsverfahren wird beispielsweise beschrieben in der Dissertation von U. Kessler mit dem Thema "Experimentelle Untersuchung und Modellierung der Überlebensrate von Milchsäurebakterien bei der thermischen Trocknung", Technische Universität München, 1993. Auf den Inhalt dieser Druckschrift wird ebenfalls in vollem Umfang Bezug genommen. Zur Durchführung des Wirbelschicht-Trocknungsverfahrens ist es von Vorteil, das zu verwendende Trägermaterial, insbesondere die Matrixkomponente, in einer Wirbelschicht vorzulegen und diese mit der Mikroorganismensuspension in der von U. Kessler beschriebenen Weise zu besprühen.

Das erfindungsgemäß am meisten bevorzugte Trocknungsverfahren ist jedoch die Sprühtrocknung. Erfindungsgemäß brauchbar sind im Wesentlichen alle bisher bekannten Sprühtrocknungstechniken. Das Sprühgut kann beispielsweise im Gleichstrom oder im Gegenstrom getrocknet werden; die Versprühung kann mittels einer Ein- oder Mehrstoffdüse oder mittels eines Zerstäuberrades erfolgen.

Erfindungsgemäß bevorzugt verwendet man Sprühgut mit einem Feststoffgehalt (nach Zugabe des Trägers) von etwa 10 bis 40, wie z. B. etwa 10 bis 25 Gew.-%.

Das erfindungsgemäße Sprühtrocknungsverfahren wird so durchgeführt, dass ein konditioniertes Trockengas mit einer Temperatur im Bereich von mehr als etwa 80 °C in die Trockenvorrichtung eingeleitet wird. Insbesondere sollte die Eintrittstemperatur im Bereich von etwa 90 bis 135 °C liegen. Besonders bevorzugt ist eine Trockentemperatur im Bereich von etwa 105 °C. Die Geschwindigkeit des Trocknungsverfahrens ist erfindungsgemäß so ausgelegt, dass die Austrittstemperatur des Trockengutes aus dem Trockner im Bereich von etwa 45 bis 75 °C, insbesondere im Bereich von etwa 50 bis 65 °C, vorzugsweise bei etwa 55 °C liegt.

Von besonderer Bedeutung für das erfindungsgemäße Verfahren ist die Verwendung von vorkonditionierter, d. h. feuchtigkeitsarmer Trockenluft. Bevorzugt verwendet man Pressluft, deren Taupunkt bei etwa -25 °C liegt.

Das erfindungsgemäße Trocknungsverfahren ist so durchzuführen, dass im Trockengut eine möglichst geringe Restfeuchte vorliegt. Bevorzugt sollte die Wasseraktivität a_{w} im Trockengut weniger als 0,4 betragen. Zur weiteren Verbesserung der Langzeit-Lagerstabilität werden erfindungsgemäß jedoch Wasseraktivitätswerte von weniger als 0,15, vorzugsweise im Bereich von etwa 0,03 bis 0,1, angestrebt. Der prozentuale Wassergehalt liegt vorzugsweise etwa bei 2 bis 3 Gew.-%. Dies erreicht man am bevorzugtesten dadurch, dass man im Anschluss an den Sprühtrocknungsschritt einen Nachtrocknungsschritt anfügt. Das Trockengut wird hierzu beispielsweise in einem Wirbelbett, vorzugsweise bei einer Temperatur im Bereich von 15 bis 50 °C, über einen Zeitraum von beispielsweise 15 Minuten bis 20 Stunden nachgetrocknet. Als Trockengas dient wiederum vorzugsweise konditionierte Pressluft bzw. konditionierter Stickstoff. Die Nachtrocknung kann aber auch durch Anlegen eines Vakuums von etwa 1 bis 50 Torr über einen Zeitraum von etwa 15 Minuten bis 20 Stunden und bei einer Temperatur von etwa 15 bis 50 °C erfolgen. Hierbei ist ein Rühren des Trockengutes beispielsweise mit Hilfe eines Schaufelrührers bevorzugt.

Anstelle der oben beschriebenen physikalischen Nachtrocknungsverfahren ist es außerdem denkbar, dem bei der Sprühtrocknung erhaltenen Trockengut spezielle Trocknungsmittel zuzusetzen. Derartige Trocknungsmittel sollten ihrerseits eine sehr niedrige Wasseraktivität, wie z. B. einen a_{w}-Wert von 0,01 oder weniger, aufweisen. Als Beispiele für geeignete Trocknungsmittel sind zu nennen anorganische Salze, wie Calciumchlorid und Natriumcarbonat, organische Polymere, wie z. B. das unter der Handelsbezeichnung Kollidion 90 F erhältliche Produkt, sowie Siliciumdioxid-haltige Trocknungsmittel, wie Kieselgel, Zeolithe, sowie Trocknungsmittel, die unter der Handelsbezeichnung Tixosil 38, Sipernat 22 S oder Aerosil 200 erhältlich sind.

Erfindungsgemäß wurde überraschenderweise festgestellt, dass trotz der relativ hohen Trocknungstemperaturen die Überlebensrate für die erfindungsgemäßen Trockenpräparate ausgezeichnete Werte von nämlich 75 % ±25 % zeigt.

Der Gehalt an lebensfähigen Mikroorganismen liegt im Bereich von etwa 4·10¹¹ bis 1 x 10¹² cfu/g Trockensubstanz. Diese Präparate werden erfindungsgemäß auch als Pulverkonzentrate bezeichnet. Da für einzelne Endanwendungen auch geringere Gehalte an lebensfähigen Mikroorganismen völlig ausreichend sind, können derartige Pulverkonzentrate deshalb gegebenenfalls durch vermengen mit weiteren inerten Trägermaterial auf die endgültige Zahl lebensfähiger Keime abgemischt werden.

### Herstellung verdichteter trockener Mikroorganismenkulturen

Die durch oben beschriebene Trocknungsverfahren erhältlichen Pulverkonzentrate besitzen gewöhnlich einen relativ hohen Staubanteil und sind somit für einzelne Anwendungen noch nicht in zufriedenstellender Weise handhabbar. Außerdem erfordern verschiedene Anwendungen eine erhöhte mechanische Stabilität der Trockenkulturen. Es ist deshalb erforderlich, die Eigenschaften der oben beschriebenen Pulverkonzentrate durch einen weiteren Verdichtungsschritt zu verbessern.

Um den Staubanteil der erfindungsgemäßen Pulverkonzentrate zu verringern, besteht die Möglichkeit, diese in herkömmlicher Weise zu einem Granulat zu agglomerieren oder unter Anwendung äußerer Kräfte zu kompaktieren oder zu tablettieren.

Die Agglomeration stellt ein allgemein bekanntes Verfahren dar und wird beispielsweise beschrieben von Schade, A. und Leuenberger, H. in Kontinuierliche Wirbelschicht-Sprühgranulation, Chemie Ingenieur Technik (1992), 64, 1016; Uhlemann, H., Herstellung pharmazeutischer Granulate in einem kombinierten Feuchtgranulations-, und Mehrkammer-Wirbelschichttrocknungsverfahren, Chemie Ingenieur Technik (1990), 62, 822; oder Rosch, M. und Probst R., Granulation in der Wirbelschicht, Verfahrenstechnik (1975), 9, 59.

Erfindungsgemäß brauchbar ist die Agglomeration mit Hilfe eines Mischers. Hierzu füllt man das oben beschriebene Pulverkonzentrat in den Mischer und düst Öl, Wasser oder eine wässrige oder alkoholische Lösung von Zuckern, Polymeren oder anderen Zusatzstoffen bei, um das Pulverkonzentrat zu agglomerieren.

Außerdem ist erfindungsgemäß brauchbar die Agglomerierung in einem Wirbelbett. Hierbei wird Pulverkonzentrat unter Gaszufuhr verwirbelt und mit einer wässrigen oder alkoholischen Lösung von Zuckern, Polymeren oder anderen Zusatzstoffen zum Agglomerataufbau besprüht. Geeignete Verfahren hierzu sind beispielsweise in der WO-A- 88/06181, in der Dissertation von U. Kessler (a.a.O) sowie von K. Fuchs in ZFL (1994) 45, 31 beschrieben. Auf die Offenbarung der obengenannten Druckschriften wird hiermit Bezug genommen.

Durch Agglomeration erhält man granulierte Mikroorganismenkulturen mit einer Korngröße im Bereich von etwa 0,1 bis etwa 4 mm, insbesondere etwa 0,3 bis 2,5 mm.

Erfindungsgemäß besonders bevorzugt ist jedoch die Herstellung trockener Mikroorganismenkulturen, welche in Form besonders stark verdichteter Partikel vorliegen. Dies erfolgt erfindungsgemäß entweder durch Tablettierung in üblichen Tablettenpressen oder unter Anwendung üblicher, mit zwei gegenläufigen Walzen ausgerüsteter Kompaktiervorrichtungen.

Vor Durchführung der Verdichtung der erfindungsgemäß erhältlichen Pulverkonzentrate werden diesem gewöhnlich ein oder mehrere Coformulantien oder Zuschlagsstoffe zugesetzt, um die Verarbeitbarkeit zum Endprodukt bzw. die Eigenschaften des Endproduktes zu modifizieren.

Zur Verbesserung der Fließfähigkeit des Pulverkonzentrates setzt man vorzugsweise ein Fließhilfsmittel zu. Als Beispiel für ein geeignetes Fließhilfsmittel sind zu nennen sprühgetrocknete Siliciumdioxidpulver, welche beispielsweise unter der Handelsbezeichnung Sipernat 50 erhältlich sind. Zur Verbesserung der Lagerstabilität der erfindungsgemäßen festen Formulierungen können außerdem übliche Antioxidanzien, wie z. B. L-Ascorbinsäure, zugesetzt werden. Außerdem können zusätzlich Trocknungsmittel des oben bereits beschriebenen Typs eingesetzt werden.

Die Wirkungsweise der erfindungsgemäßen Kulturen wird deutlich verbessert, wenn Vorkehrungen getroffen werden, die, nach Applikation der Kultur, zu einem schnellen Zerfall der Kornstruktur und somit zu einer raschen Freisetzung der Mikroorganismen führen. Eine Möglichkeit, dies zu erreichen, besteht in der Zugabe einer gut wasserlöslichen und damit den Zerfall der Kornstruktur beschleunigenden Komponente. Als geeignete Verbindungen sind beispielsweise zu nennen Polyethylenglykole, welche z. B. unter der Handelsbezeichnung Pluriol E erhältlich sind.

Eine andere, erfindungsgemäß besonders bevorzugte Feststofformulierung umfasst einen sogenannten Brausezusatz. Hierbei handelt es sich um eine gasfreisetzende Komponente, insbesondere eine CO₂-Quelle, wie z. B. um ein Erdalkalihydrogencarbonat, vorzugsweise Natriumhydrogencarbonat oder um Ammoniumhydrogencarbonat; sowie um eine Säurekomponente, vorzugsweise ausgewählt unter Citronensäure, Ascorbinsäure oder Äpfelsäure. Dieser Brausezusatz bewirkt in Gegenwart von Feuchtigkeit eine spontane Gasentwicklung unter Zerfall der Kornstruktur und schneller Freisetzung der Mikroorganismenkeime.

Insbesondere für die Herstellung stark verdichteter, kompaktierter oder tablettierter Mikroorganismenkulturen empfiehlt sich die Zugabe von Kompaktierungs- oder Tablettierungshilfsmitteln. Es wurde nämlich erfindungsgemäß überraschenderweise festgestellt, dass durch Zugabe solcher Kompaktierungshilfsmittel die während der Kompaktierung auf die Mikroorganismen einwirkenden Drücke verringert und somit die Überlebensrate der Keime deutlich verbessert wird. Als Beispiele für geeignete Kompaktierhilfsmittel sind zu nennen mikrokristalline Cellulose, Zucker sowie Mischungen davon. Als konkrete Beispiele für mikrokristalline Cellulose sind Produkte zu nennen, welche unter den Handelsnamen Avicel, Arbocel und Vivapur kommerziell erhältlich sind. Beispiele für geeignete Zucker sind Maltose, Maltodextrin sowie Lactosepräparate, welche unter den Handelsnamen Granulac, Tablettose oder FloLac erhältlich sind. Als Beispiel für ein geeignetes Cellulose/Zuckermischprodukt ist zu nennen das Handelspräparat Cellactose. Als weiteres geeignetes Tablettierhilfsmittel zu nennen ist ein mit PVP granuliertes Lactosepräparat, erhältlich unter dem Handelsnamen Ludipreß.

Weitere geeignete Zusätze sind Polyethylenglykole (Mw 100 bis 10000) welche auf die in der Matrix eingebetteten Zellen eine stabilisierende Wirkung besitzen können.

Beiliegende Figur 1 zeigt ein Fließdiagramm für die erfindungsgemäße Weiterverarbeitung der Pulverkonzentrate zu einem erfindungsgemäßen kompaktierten Produkt. Pulverkonzentrat PK wird im Mischer M1 mit den Coformulantien oder Zuschlagstoffen ZU vermischt, gelangt von dort aus in einen vorratsbehälter B1, welcher den Kompaktor A1 speist. Das vom Kompaktor austretende Produktband wird in den Mahlwerken Z1 und Z2 vorzerkleinert bzw. endzerkleinert und im Sieb F1 wird Produkt PR von Staubanteilen mit einer Korngröße von weniger als 0,3 mm abgetrennt. Dieses wird als Rückgut RÜ dem Mischer M1 zugeführt. Das Produkt PR mit einer Korngröße von 0,3 mm oder mehr, wie z. B. 0,3 bis 1,5 mm, gelangt zur Abfüllung oder wird gegebenenfalls einer Weiterbehandlung, wie z. B. einem Coatingprozess, unterzogen.

Geeignete Coatingmaterialien, welche vorzugsweise den Zutritt von Feuchtigkeit zum Trockenpräparat zusätzlich erschweren sollen, sind beispielsweise alkoholische Lösungen von PVP, insbesondere eines PVP-Produktes, das unter der Handelsbezeichnung Kollidon VA64 im Handel erhältlich ist. Ein anderes brauchbares Coatingsystem stellt ein Gemisch aus Shellac und Kollidon 25 oder 30 dar, welches mit Titandioxid und Talg supplementiert ist und ebenfalls in alkoholischer Lösung vorliegt.

Um die Zellzahl gegebenenfalls weiter zu verringern, kann ein auf diese Weise erhaltenes gecoatetes oder nicht-gecoatetes Produkt beispielsweise mit Kalk oder einem anderen geeigneten mineralischen Zusatz abgemischt werden.

### Ausführungsbeispiele

In den folgenden Beispielen verwendete Analysenmethoden:

### a) Bestimmung der Zellzahl:

Zellzahlbestimmungen wurden in der üblichen Art durch serielle Verdünnung mit steriler 0,9%iger NaCl-Lösung und anschließender Plattierung auf MRS-Agar (Difco Laboratories) durchgeführt. Kolonienbildende Einheiten ("cfu") wurden nach 48-stündiger Bebrütung bei 37°C gezählt. Es wurden nur solche Platten berücksichtigt, die mindestens 30 und höchstens 300 Kolonien enthielten. In der Regel wurden drei Platten pro Stufe ausgewertet und der Mittelwert gebildet.

Die spezifische Zellzahl einer Probe wurde rechnerisch ermittelt, indem die Zellzahl pro Gramm Probe durch den relativen Anteil des Trockengewichts der Probe geteilt wurde.

### b) Bestimmung der Überlebensrate bei Trocknung:

Die Überlebensrate bei der Trocknung wurde errechnet aus der spezifischen Zellzahl der Probe vor der Trocknung geteilt durch die spezifische Zellzahl nach der Trocknung. Sie wurde stets in Prozent ausgedrückt.

### c) Bestimmung der Lagerstabilität:

Um die Lagerstabilität einer getrockneten Probe zu bestimmen, wurde die spezifische Zellzahl der getrockneten Probe unmittelbar nach der Trocknung bestimmt (Tag_{O}). Das getrocknete Zellmaterial wurde unter Luft in einem lichtundurchlässigen, dicht verschlossenen Gefäß bei Raumtemperatur (21°C ±2 °C) über längere Zeiträume gelagert. In regelmäßigen Abständen wurde die spezifische Zellzahl erneut ermittelt (Tag_{N}). Die Lagerstabilität wurde errechnet aus dem Quotienten der spezifischen Zellzahl Tag_{N}/spezifischer Zellzahl Tag_{O}.

Lag die spezifische Zellzahl nach der Trocknung z. B. bei 5·10¹¹ cfu/TS und nach 8 wochen Lagerung bei 4·10¹¹ cfu/g TS, so betrug die Lagerstabilität 80 % des Ausgangswertes.

### d) Messung des Feuchtegehaltes:

Elektronisches Feuchtebestimmungsgerät HR 73 der Fa. Mettler Durchführung: ca. 2 g Pulver werden auf dem Messschälchen des Gerätes verteilt. Gemessen wird bei 105°C Trocknungstemperatur bis zur Gewichtskonstanz (Abbruchkriterium: max. 1 mg Gewichtsverlust in 50 sec).

### e) Messung der Wasseraktivität:

Gerät Hygroskop DT der Fa. Rotronic AG, Zürich, Schweiz
Das Produkt wird in die Messaufnahmeschale gegeben und diese in die auf 25°C thermostatisierte Messkammer gestellt. Nach dem Schließen der Messkammer und einer Äquilibrierungszeit von 20 Minuten wird der Gerätemesswert abgelesen.

### f) DSC-Messung zur Bestimmung der Glasübergangstemperatur Tg:

Gerät TA4000 der Fa. Mettler
Einwaage ca. 15 mg, Heizrate 20 °C/min, Proben wurden während der Messung mit einem Stickstoffstrom umspült.
DSC = Differential Scanning Calorimetry

### Beispiele zur Kultivierung von Mikroorganismen

### Beispiel K1: Batch Fermentation 10 Liter Maßstab

10 l eines Fermentationsmediums, welches folgende Bestandteile enthielt, wurden in einen 14 1-Fermenter gegeben und bei 121 °C für 30 Minuten sterilisiert:

| | |
|---|---|
| Glucosemonohydrat | 550,0 g |
| 50 % Hefeextraktsuspension (pH 4,5 mit Phosphorsäure) | 750,0 g |
| Tween 80® | 10,0 g |
| MgSO₄ ∗ 7 H₂O | 5,0 g |
| MnSO₄ ∗ 1 H₂O | 0,5 g |

Nach der Sterilisation wurde das Medium mit steriler 25%iger Natronlauge auf pH 5,8 bei 37 °C eingestellt und das Medium mit einem sanften Strom sterilen Stickstoffs überlagert. Der Fermenter wurde mit 150 UpM gerührt.

Der Fermenter wurde dann mit 100 ml einer Lactobacillus plantarum Vorkultur (BASF Stamm LU 3244) beimpft, welche zuvor 16 h bei 37 °C in MRS-Nährmedium (Difco Laboratories) gewachsen war. Der pH-Wert der Kultur wurde kontinuierlich mit 25%iger Natronlauge auf 6,2 geregelt.

Der Verlauf der Fermentation wurde anhand des Natronlaugeverbrauchs verfolgt. Sobald keine Natronlauge mehr verbraucht wurde (Gesamtverbrauch 890 g), wurde die gesamte Fermentationsbrühe abgelassen und bei 8 °C zentrifugiert. Die Biomasse wurde in etwa 600 g Überstand resuspendiert und auf genau 1000 g mit Überstand aufgefüllt. Der Trockengewichtsanteil wurde mit Hilfe einer Infrarot-Trockenwaage bestimmt (105 °C bis Gewichtskonstanz). Der Feststoffgehalt dieser Suspension betrug 15 %.

### Beispiel K2: Batch Fermentation 200 Liter Maßstab

180 l eines Fermentationsmediums, welches folgende Bestandteile enthielt, wurden in einen 200 l-Fermenter gegeben und bei 121 °C für 30 Minuten sterilisiert:

| | |
|---|---|
| Glucosemonohydrat | 11 kg |
| 50 % Hefeextraktsuspension | 15 kg |
| Tween 80® | 200 g |
| MgSO₄ ∗ 7 H₂O | 200 g |
| MnSO₄ ∗ 1 H₂O | 10 g |

Nach der Sterilisation wurde das Medium mit steriler 25%iger Natronlauge auf pH 5,8 bei 37 °C eingestellt und das Medium mit einem sanften Strom sterilen Stickstoffs überlagert.

Der Fermenter wurde dann mit 2000 ml einer Lactobacillus plantarum Vorkultur (3244) beimpft, welche zuvor 24 h bei 30 °C in MRS-Nährmedium gewachsen war. Der pH-Wert der Kultur wurde kontinuierlich mit 25%iger Natronlauge geregelt.

Der Verlauf der Fermentation wurde anhand des Natronlaugeverbrauchs verfolgt. Insgesamt wurde 16,43 kg 25%iger NaOH verbraucht. Sobald keine Natronlauge mehr verbraucht wurde, wurde die gesamte Fermentationsbrühe abgelassen und bei 8 °C mit Hilfe eines kontinuierlichen Separators geerntet. Das Gewicht der geernteten Biomasse betrug nach der Zentrifugation 20 kg. Der Feststoffgehalt dieser Suspension betrug 12,3 %. Die Zellzahl der Suspension betrug 1,04·10¹¹ cfu/g Suspension. Die spezifische Zellzahl betrug 8,45·10¹¹ cfu/g Trockensubstanz (TS).

### Beispiel K3: Batch Fermentation mit Temperaturschock

Es wurde eine Fermentation analog Beispiel 2 durchgeführt. Bei einem Natronlaugenverbrauch entsprechend 90 % des zu erwartenden Wertes wurde die Fermentertemperatur auf 44 °C erhöht und gehalten, bis der gesamte vorgelegte Zucker verbraucht war. Anschließend wurden die Zellen, wie in Beispiel K2 beschrieben, geerntet. Die Zellzahl der Fermentationsbrühe betrug 1,8·10¹¹ cfu/g bei einem Feststoffgehalt von 21,17 %. Dies entspricht einer spezifischen Zellzahl von 8,5·10¹¹ cfu/g TS.

### Beispiel K4: Kontinuierliche Fermentation

10 l eines Fermentationsmediums mit folgender Zusammensetzung wurden in einen 14 1-Fermenter eingefüllt und 30 Minuten bei 121 °C sterilisiert (Produktionsfermenter):

| | |
|---|---|
| Glucosemonohydrat | 400,0 g |
| 50 % Hefeextraktsuspension (pH 4,5 mit Phosphorsäure) | 500,0 g |
| KH₂PO₄ | 30,0 g |
| Zitronensäuremonohydrat | 21,0 g |
| Tween 80® | 10,0 g |
| MgSO₄ ∗ 7 H₂O | 5,0 g |
| MnSO₄ ∗ 1 H₂O | 1, 7 g |
| (NH₄)₂Fe(SO₄)₂ ∗ 6 H₂O | 0,4 g |

In einem zweiten Fermenter mit einem Gesamtvolumen von 3 000 l wurden 2 000 l des gleichen Mediums eingefüllt und sterilisiert (Vorratsfermenter). Beide Fermenter wurden mit einer sterilisierbaren Leitung verbunden. Über ein Zwischengefäß, welches auf einer Waage stand, wurden dann mittels einer automatischen Steuerung jeweils 3 l frischen Mediums pro Stunde in den Produktionsfermenter gepumpt. Die Temperatur des Produktionsfermenters wurde auf 37 °C geregelt. Der pH-Wert wurde mit 25%iger NaOH auf 5,8 geregelt. Der Fermenter wurde mit 150 UpM gerührt und mit 0,1 VVM Stickstoff überlagert.

Über eine zweite Pumpe wurden jeweils 3 l Medium pro Stunde kontinuierlich abgezogen und in einem auf 0 bis 4 °C vorgekühlten Sammelbehälter aus Edelstahl gesammelt. Die Konzentration an Biomasse wurde turbidometrisch bestimmt und betrug 3,5 g/l. Die Glucosekonzentration im Auslauf des Produktionsfermenters betrug nach der anfänglichen Anwachsphase zu allen Zeiten 0 g/l. Die Zellzahl der Fermentationsbrühe betrug 1,48·10¹⁰ cfu/g Brühe. Der Trockengewichtsanteil der Fermentationsbrühe betrug 6,89 %, entsprechend 217 g TS. Die spezifische Zellzahl betrug 2,15·10¹¹ cfu/g TS.

### Beispiel K5: Ernte der Zellen mit Waschschritt zur Entfernung von Natriumlactat

72 l Fermenteraustrag aus Beispiel K4 wurden kontinuierlich mit Hilfe eines handelsüblichen Separators bei 8 °C geerntet. Es wurden etwa 7 kg Zellsuspension gewonnen. Zu diesen wurde eine Waschlösung hinzugegeben, welche auf 40 l VE-Wasser, 450 g NaCl und 136 g KH₂PO₄ enthielt. Der pH-Wert der Waschlösung war zuvor mit 25%iger Natronlauge auf 7,0 eingestellt worden. Die etwa 50 l resuspendierter Zellen wurden erneut separiert. Es wurden 3 160 g konzentrierter, gewaschener Zellsuspension gewonnen. Der Feststoffgehalt der Suspension betrug 9,97 %. Die Zellzahl betrug 2,49·10¹¹ cfu/g Suspension. Die spezifische Zellzahl betrug 2,5·10¹² cfu/g TS.

Diese gewaschene Zellsuspension war praktisch frei von Natriumlactat. Die Biomassekonzentration wurde turbidometrisch bestimmt. Sie betrug 80 g/l.

### Beispiele für Herstellung erfindungsgemäßer Pulverkonzentrate durch Sprühtrocknung

Die im folgenden Abschnitt beschriebenen Sprühtrocknungsversuche zur Herstellung erfindungsgemäßer Pulverkonzentrate werden in einen Labor-Sprühtrockner Typ Niro Minor der Fa. Niro, Kopenhagen, Dänemark, durchgeführt. Die sprühfertige Bakteriensuspension wird über eine Zweistoffdüse zusammen mit vorkonditionierter, erhitzter Pressluft im Gleichstrom in den Trockenturm der Anlage eingesprüht, das getrocknete Produkt wird mit Hilfe eines Zyklons von der Luft abgetrennt und gesammelt.

### Beispiel S1

zur Herstellung einer Coformulantien-Lösung werden 200 ml VE-Wasser (vollentsalztes Wasser) auf 60°C erwärmt. Darin werden 150 g Süssmolkepulver, 7,5 g NaCl, 3,8 g KH₂PO₄, 3,8 g Zitronensäure und 3, 8 g l-Ascorbinsäure gelöst, mit 40 %igem wässrigem NaOH auf pH 7 eingestellt und mit VE-Wasser auf 400 g Gesamtmasse aufgefüllt. Diese Lösung wird auf 5 °C abgekühlt.

200 ml gewaschenes, d. h. im Wesentlichen natriumlactatfreies Ferment-Zentrifugat (hergestellt analog Beispiel K5)(12,7 % Feststoffgehalt (F.G.)) werden im Eisbad bei einer Temperatur von 5 °C vorgelegt und unter Rühren mit 400 g Coformulantien-Lösung, abgekühlt auf 5 °C, versetzt. Die Mischung aus Ferment-Zentrifugat und Coformulantien wird 30 Minuten bei 500 U/min mittels Magnetrührer unter Eisbadkühlung nachgerührt. Anschließend wird die Mischung mittels Sprühtrocknung (Gerät Niro Minor) in ein Pulverkonzentrat A überführt, das am Zyklon abgeschieden wird. Dabei wird die Vorlage, aus der die Mischung zudosiert wird, auf 4 °C gekühlt, die Eintrittstemperatur beträgt 105 bis 110 °C, die Austrittstemperatur beträgt 53,5 bis 55,5 °C. Verwendet wird eine Zweistoffdüse, wobei konditionierte Luft (Taupunkt -25 °C) bei 4 bar zum Versprühen der Mischung aus Ferment-Zentrifugat und Coformulantien verwendet wird.

Das Pulverkonzentrat A wird ein einem mit Stickstoff (Taupunkt = -40°C) betriebenen Wirbelbett 2 Stunden bei Raumtemperatur nachgetrocknet, wobei man Pulverkonzentrat B erhält.

### Charakterisierungen:

| | |
|---|---|
| Sprühfertige Mischung: | 35 % F.G., 2,84·10¹¹ cfu/g Trockensubstanz |
| Pulverkonzentrat A: | Wasseraktivität a_{w} = 0,135 |
| Pulverkonzentrat B: | wasseraktivität a_{w} = 0,076, Feuchtegehalt 3,4 %, T_{g} aus DSC-Messung: 54°C, 1,98·10¹¹ cfu/g Trockensubstanz (entspricht 70 % Überlebensrate in der Trocknung) |
| Lagerstudie Pulverkonzentrat B: | cfu-Zahlen bei Raumtemperaturlagerung in unter Raumluft verschlossenen Gefäßen: 2,0·10¹¹ cfu/g Trockensubstanz (100 %) nach 30 Tagen |

### Beispiel S2

Zur Herstellung einer Coformulantien-Lösung werden 200 ml VE-Wasser auf 70°C erwärmt. Darin werden 75 g Maltodextrin (Glucidex IT6, Fa. Roquette), 75 g Lactose, 7,5 g NaCl, 3,8 g KH₂PO₄, 3,8 g Zitronensäure und 3,8 g l-Ascorbinsäure gelöst, mit 40 %igem wässrigem NaOH auf pH 7 eingestellt und mit VE-Wasser auf 400 g Gesamtmasse aufgefüllt. Diese Lösung wird auf 5 °C abgekühlt.

200 ml gewaschenes, d. h. im Wesentlichen natriumlactatfreies Ferment-Zentrifugat (16,5 % F.G.; hergestellt analog Beispiel K5) bei 5 °C unter Rühren in 400 g Coformulantien-Lösung, abgekühlt auf 5°C, eingemischt. Die Mischung wird 30 Minuten bei 250 U/min mittels Magnetrührer unter Eisbadkühlung gerührt. Danach werden 101 ml eines nach EP-A-0 479 066 (BASF) hergestellten Solubilisates aus 25 % Tween 80, 5 % β-Carotin und 2 % α-Tocopherol zugegeben und unter Eisbadkühlung 10 Minuten nachgerührt. Anschließend wird diese Mischung mittels Sprühtrocknung, wie in Beispiel S1 beschrieben, in ein Pulverkonzentrat A überführt (Eintrittstemperatur 105°C, Austrittstemperatur 54 bis 55°C). Das Pulverkonzentrat A wird nicht nachgetrocknet.

### Charakterisierungen:

| | |
|---|---|
| Sprühfertige Mischung: | 29% F.G., 3,84·10¹¹ cfu/g Trockensubstanz |
| Pulverkonzentrat A: | Wasseraktivität a_{w} = 0,065, Feuchtegehalt 2,8 %, T_{g} aus DSC-Messung: 61°C, 2,22·10¹¹ cfu/g Trockensubstanz (entspricht 58 % Überlebensrate in der Trocknung) |
| Lagerstudie Pulverkonzentrat A: | cfu-Zahlen bei Raumtemperaturlagerung in unter Raumluft verschlossenen Gefäßen: 1,9·10¹¹ cfu/g Trockensubstanz (86 %) nach 30 Tagen |

### Beispiel S3

400 ml ungewaschenes, d. h. natriumlactathaltiges Ferment-Zentrifugat (hergestellt analog Beispiel K4)(14,3 % F.G.) werden im Eisbad bei einer Temperatur von 5 °C vorgelegt. Unter Rühren bei 700 U/min mittels Magnetrührer gerührt werden nun 57,2 g Glucidex IT6, 8,6 g l-Ascorbinsäure und 5,7 % Natriumglutamat als Feststoffe in das gekühlte Ferment-Zentrifugat eingerührt. Der pH wird mit 40 %igem wässrigem NaOH auf pH 7 eingestellt. Die Mischung aus Ferment-Zentrifugat und Coformulantien wird 30 Minuten bei 500 U/min mittels Magnetrührer bei ca. 3 °C unter Eisbadkühlung nachgerührt. Anschließend wird die Mischung mittels Sprühtrocknung, wie in Beispiel S1 beschrieben, in ein Pulverkonzentrat A überführt (Eintrittstemperatur 105°C; Austrittstemperatur 54,5 bis 55,5 °C).

Das Pulverkonzentrat A wird in einem mit Stickstoff betriebenen Wirbelbett 2 Stunden bei Raumtemperatur nachgetrocknet, wobei man ein Pulverkonzentrat B erhält.

### Charakterisierungen:

| | |
|---|---|
| Sprühfertige Mischung: | 27 % F.G., 4,65·10¹¹ cfu/g Trockensubstanz |
| Pulverkonzentrat A: | Wasseraktivität a_{w} = 0,197 |
| Pulverkonzentrat B: | Wasseraktivität a_{w} = 0,072, Feuchtegehalt 3,8 %, T_{g} aus DSC-Messung: 52 °C, 4,64·10¹¹ cfu/g Trockensubstanz (entspricht 100 % Überlebensrate in der Trocknung) |
| Lagerstudie Pulverkonzentrat B: | cfu-Zahlen bei Raumtemperaturlagerung in unter Raumluft verschlossenen Gefäßen: 4,1·10¹¹ cfu/g Trockensubstanz (88 %) nach 28 Tagen |

### Beispiel S4

215 ml gewaschenes, d. h. im Wesentlichen natriumlactatfreies Ferment-Zentrifugat (hergestellt analog Beispiel K5) (14,5 % F.G.) werden im Eisbad bei einer Temperatur von 5 °C vorgelegt. Unter Rühren bei 700 U/min mittels Magnetrührer werden nun 31,2 g Glucidex IT6, 4,7 g Ascorbinsäure und 3,1 % Natriumglutamat als Feststoffe in das gekühlte Ferment-Zentrifugat eingerührt. Der pH wird mit 40 %igem wässrigem NaOH auf pH 7 eingestellt. Die Mischung aus Ferment-Zentrifugat und Coformulantien wird 30 Minuten bei 500 U/min mittels Magnetrührer unter Eisbadkühlung nachgerührt. Anschließend wird die Mischung mittels Sprühtrocknung, wie in Beispiel S1 beschrieben, in ein Pulverkonzentrat A überführt (Eintrittstemperatur 105 °C; Austrittstemperatur 54,5 bis 55,5°C).

Das Pulverkonzentrat A wird in einem mit Stickstoff betriebenen Wirbelbett 2 Stunden bei Raumtemperatur nachgetrocknet, wobei man Pulverkonzentrat B erhält.

### Charakterisierungen:

| | |
|---|---|
| Sprühfertige Mischung: | 28 % F.G., 8,76·10¹¹ cfu/g Trockensubstanz |
| Pulverkonzentrat B: | Wasseraktivität a_{w} = 0,044, Feuchtegehalt 3,8 %, T_{g} aus DSC-Messung: 48 °C, 7,17·10¹¹ cfu/g Trockensubstanz (entspricht 82 % Überlebensrate in der Trocknung) |
| Lagerstudie Pulverkonzentrat B: | cfu-Zahlen bei Raumtemperaturlagerung in unter Raumluft verschlossenen Gefäßen: 3,7·10¹¹ cfu/g Trockensubstanz (52 %) nach 27 Tagen |

### Beispiel S5

Zur Herstellung einer Coformulantien-Lösung 1 werden 40 ml VE-Wasser vorgelegt und darin 33,3 g Lactose und 6,3 g Pepton gelöst, mit VE-Wasser auf 83 g Gesamtmasse aufgefüllt und mit 40 %igem wässrigem NaOH auf pH 7 eingestellt. Zur Herstellung einer Coformulantien-Lösung 2 werden 40 ml VE-Wasser vorgelegt und darin 33,3 g Glucose-1-hydrat und 5,4 g Zitronensäure gelöst, mit VE-Wasser auf 83 g Gesamtmasse aufgefüllt und mit 40 %igem wässrigem NaOH auf pH 7 eingestellt. Diese Lösungen 1 und 2 werden auf 5 °C abgekühlt.

200 ml gewaschenes, d. h. im Wesentlichen natriumlactatfreies Ferment-Zentrifugat (hergestellt analog Beispiel K5)(12,7 % F.G.) werden im Eisbad bei ca. 4 °C unter Rühren mit 83 g der abgekühlten Coformulantien-Lösung 1 vermischt. Die Mischung wird 30 Minuten unter Eisbadkühlung gerührt. Danach werden unter Rühren 83 g der abgekühlten Coformulantien-Lösung 2 zugegeben und unter Eisbadkühlung 30 Minuten nachgerührt. Anschließend wird diese Mischung mittels Sprühtrocknung, wie in Beispiel S1 beschrieben, in ein Pulverkonzentrat A überführt (Eintrittstemperatur 105°C; Austrittstemperatur 55°C).

Das Pulverkonzentrat A wird in einem mit Stickstoff betriebenen wirbelbett 2 Stunden bei Raumtemperatur nachgetrocknet, wobei man Pulverkonzentrat B erhält.

### Charakterisierungen:

| | |
|---|---|
| Sprühfertige Mischung: | 29 % F.G., 7,30·10¹¹ cfu/g Trockensubstanz |
| Pulverkonzentrat B: | Wasseraktivität a_{w} = 0,139, Feuchtegehalt 3,7 %, T_{g} aus DSC-Messung: 45°C, 5,06·10¹¹ cfu/g Trockensubstanz (entspricht 69 % Überlebensrate in der Trocknung) |
| Lagerstudie Pulver-konzentrat B: | cfu-Zahlen bei Raumtemperaturlagerung in unter Raumluft verschlossenen Gefäßen: 4,8·10¹¹ cfu/g Trockensubstanz (95 %) nach 21 Tagen |

### Beispiel S6

Die Herstellung der sprühfertigen Mischungen erfolgte analog zu Beispiel S3. Verwendet wurden hier aber zwei unterschiedliche Ferment-Zentrifugate:

Beispiel S6a: Batch-Fermentation, wobei das Ferment gegen Ende der Fermentation für 40 Minuten auf 4 °C gekühlt wurde.

Das in der Sprühtrocknung gemäß Beispiel S1 (Eintrittstemperatur 107 bis 111°C; Austrittstemperatur 58 bis 61°C) erhaltene Pulverkonzentrat A wurde nicht nachgetrocknet.

### Charakterisierungen:

| | |
|---|---|
| Sprühfertige Mischung: | 3,68·10¹¹ cfu/g Trockensubstanz |
| Pulverkonzentrat A: | 0,76·10¹¹ cfu/g Trockensubstanz (entspricht 21 % Überlebensrate in der Trocknung) |

Beispiel S6b: Batch-Fermentation, wobei das Ferment gegen Ende der Fermentation auf 44°C erhitzt wurde. In diesem Beispiel wurde die sprühfertige Mischung geteilt. In einem ersten Versuch wurde das Vorlagegefäß wie in den Beispielen S1 bis S5 und S6a auf 4 °C thermostatisiert. In einem zweiten Versuch wurde das Vorlagegefäß auf 20°C thermostatisiert.

Die in der Sprühtrocknungen gemäß Beispiel S1 (Eintrittstemperatur 103 bis 110 °C; Austrittstemperatur 59 bis 61 °C) erhaltenen Pulverkonzentrate A wurde nicht nachgetrocknet.

| Charakterisierungen für Vorlage bei 4 °C: | |
|---|---|
| Sprühfertige Mischung: | 3,53·10¹¹ cfu/g Trockensubstanz |
| Pulverkonzentrat A: | 2,36·10¹¹ cfu/g Trockensubstanz (entspricht 67 % Überlebensrate in der Trocknung) |

| Charakterisierungen für Vorlage bei 20°C: | |
|---|---|
| Sprühfertige Mischung: | 3,53·10¹¹ cfu/g Trockensubstanz 1,48·10¹¹ cfu/g |
| Pulverkonzentrat A: | Trockensubstanz (entspricht 42 % Überlebensrate in der Trocknung) |

### Formulierungsbeispiele

Entsprechend den im Folgenden angegebenen Rezepturen wurden Trokkenmischungen von erfindungsgemäßen Pulverkonzentraten hergestellt und zu kompaktierten Starterkulturpräparaten verarbeitet:

Wenn keine anderen Angaben gemacht wurden, wurde als Trennmittel Leucin und als Fließhilfsmittel Sipernat 50S (sprühgetrocknetes Siliziumdioxid) verwendet.

Die Einzelkomponenten der Präparate werden zunächst miteinander vermischt. Hierzu verwendet man beispielsweise einen Pflugscharmischer (Typ Lö 20 der Fa. Lödige). Die auf diese Weise erhaltene Trockenmischung wird in einem Kompaktor kompaktiert. Beispielsweise kann man hierzu einen Laborkompaktor verwenden, der eine Presskraft von 14 kN/cm² aufbringt (z. B. Laborkompaktor L 200 der Fa. Bepex). Das aus dem Kompaktor austretende Produktband wird anschließend auf eine Teilchengröße von ≤ 1,25 mm zerkleinert. Das Rohgranulat wird zur Abtrennung von Feingut einer Teilchengröße von ≤ 0,3 mm gesiebt. Die Nutzgutausbeute beträgt etwa 50 bis 60 % des eingesetzten Materials.

### Beispiel F1: Herstellung eines Brausekompaktats zur Verwendung als Starterkultur für Silage

| Präparat A: | |
|---|---|
| Pulverkonzentrat (gemäß Beispiel S2) | 200,0 g |
| Zitronensäure, wasserfrei | 95,0 g |
| NaHCO₃ | 95,0 g |
| PEG (M_{w} < 400) | 8,0 g |
| Fließhilfsmittel | 2,0 g |

| Präparat B: | |
|---|---|
| Pulverkonzentrat (gemäß Beispiel S2) | 100,0 g |
| Ascorbinsäure, Pulver | 47,5 g |
| NaHCO₃ | 47,5 g |
| PEG (M_{w} < 400) | 4,0 g |
| Fließhilfsmittel | 1,0 g |

| Präparat C: | |
|---|---|
| Pulverkonzentrat (gemäß Beispiel S2) | 100,0 g |
| Äpfelsäure | 47,5 g |
| NaHCO₃ | 47,5 g |
| PEG (M_{w} < 400) | 4,0 g |
| Fließhilfsmittel | 1,0 g |

| Präparat D: | |
|---|---|
| Pulverkonzentrat (gemäß Beispiel S2) | 100,0 g |
| Zeolith A (Wessalith P) | 20,0 g |
| Ascorbinsäure, Pulver | 37,0 g |
| NaHCO₃ | 36,8 g |
| Trennmittel | 3,0 g |
| Fließhilfsmittel | 3,0 g |

### Beispiel F2: Herstellung eines schnelllöslichen Kompaktats ohne Brausezusatz

| | |
|---|---|
| Pulverkonzentrat (gemäß Beispiel S2) | 100,0 g |
| wasserlösl. Tensid (Pluriol EL 500) | 90,0 g |
| Trennmittel | 7,0 g |
| Fließhilfsmittel | 3,0 g |

### Beispiel F3: Herstellung eines Kompaktats

| | |
|---|---|
| Pulverkonzentrat (gemäß Beispiel S5) | 100,0 g |
| Kompaktierhilfsmittel¹⁾ | 90,0 g |
| Trennmittel | 7,0 g |
| Fließhilfsmittel | 3,0 g |

| | |
|---|---|
| 1) ausgewählt unter: Avicel PH 102, Vivapur 105, FlowLac, Maltex 20, Cellactose oder Gemische davon | |

### Beispiel F4: Herstellung stabilisierter Kompaktate

### Basisrezeptur:

| | |
|---|---|
| Pulverkonzentrat (gemäß Beispiel S5) | 100,0 g |
| Kompaktierhilfsmittel | 50,0 g |
| Stabilisator | vgl. Tabelle I |
| Trennmittel | 7,0 g |
| Fließhilfsmittel | 3,0 g |

**Tabelle I**

| Stabilisator | Komponente | Menge (g) |
|---|---|---|
| A | Zeolith A | 40 |
| B | PEG 4000 | 40 |
| C | Ascorbinsäure¹) | 40 |
| D | PEG 4000 | 20 |
| | Ascorbinsäure | 20 |
| E | Zeolith A | 20 |
| | Ascorbinsäure | 20 |
| F | Zeolith A | 20 |
| | Ascorbinsäure | 3 |
| | PEG 4000 | 17 |
| G | Zeolith A | 10 |
| | Ascorbinsäure | 1,5 |
| | PEG 4000 | 8,5 |
| H | Zeolith A | 7 |
| | Ascorbinsäure | 1 |
| | PEG 4000 | 6 |

| | | |
|---|---|---|
| 1) jeweils 1-Ascorbinsäure | | |

## Patentansprüche

1. Trockene Mikroorganismenkultur, enthaltend wenigstens eine Mikroorganismen-Spezies in geträgerter Form, **dadurch gekennzeichnet, dass** die Kultur in Form von Partikeln vorliegt, welche
a) eine Partikelgröße von wenigstens etwa 0,1 mm aufweisen;
b) etwa 10¹⁰ bis 4·10¹¹ cfu/g wenigstens einer Mikroorganismen-Spezies enthalten; und
c) verdichtet sind.

2. Mikroorganismenkultur nach Anspruch 1, **dadurch gekennzeichnet, dass** die Partikel mit einer Linienkraft von etwa 5 bis 15 kN/cm kompaktiert oder einem Druck von etwa 90 bis 160 MPa tablettiert wurden.

3. Mikroorganismenkultur nach Anspruch 1 oder 2, **dadurch gekennzeichnet, dass** sie kompaktiertes Brechgut in Form von verdichteten, zerkleinerten Partikeln mit einem Durchmesser von etwa 0,1 mm bis etwa 2 mm umfasst.

4. Mikroorganismenkultur nach Anspruch 1, **dadurch gekennzeichnet, dass** die verdichteten Partikel Tabletten mit einem Durchmesser von etwa 2 bis 50 mm und einem Verhältnis von Durchmesser zu Dicke von etwa 1:0,1 bis etwa 10:1 umfassen.

5. Mikroorganismenkultur nach einem der vorhergehenden Ansprüche, **dadurch gekennzeichnet, dass** sie als weitere Komponente einen Brausezusatz umfasst.

6. Mikroorganismenkultur nach einem der vorhergehenden Ansprüche, **dadurch gekennzeichnet, dass** sie als Träger wenigstens ein Matrixmaterial zur Einbettung der Mikroorganismenzellen und gegebenenfalls wenigstens ein weiteres, die Zellen stabilisierendes Additiv umfasst.

7. Mikroorganismenkultur nach einem der vorhergehenden Ansprüche, **dadurch gekennzeichnet, dass** sie wenigstens eine Milchsäure produzierende Bakterienspezies enthält.

8. Mikroorganismenkultur nach Anspruch 7, **dadurch gekennzeichnet, dass** die Bakterienspezies ausgewählt ist unter Bakterien der Gattung Lactobacillus sp.

9. Pulverkonzentrat einer Mikroorganismenkultur, enthaltend 4·10¹¹ bis 1·10¹² cfu/g wenigstens einer Mikroorganismen-Spezies und einen Träger.

10. Pulverkonzentrat nach Anspruch 9, **dadurch gekennzeichnet, dass** es eine Wasseraktivität a_{w} von weniger als 0,4 aufweist.

11. Verfahren zur Herstellung eines Pulverkonzentrats nach Anspruch 9 oder 10, **dadurch gekennzeichnet, dass** man
a) in einer wenigstens eine Mikroorganismen-Spezies enthaltende Flüssigkeit wenigstens eine zur Ausbildung eines Trägers geeignete Substanz löst oder suspendiert,
b) das so erhaltene Gemisch in einem Sprühtrockner trocknet, wobei man zur Sprühtrocknung ein konditioniertes, getrocknetes Gas mit einem Taupunkt von etwa -10 bis etwa -50 °C, welches auf eine Temperatur im Bereich von mehr als etwa 80 °C erhitzt wird, verwendet, und
c) das Trockengut aus dem Sprühtrockner entfernt, wobei dieses eine Austrittstemperatur von etwa 45 bis 75 °C aufweist.

12. Verfahren nach Anspruch 11, **dadurch gekennzeichnet, dass** man in einer weiteren Stufe d) das Trockengut einer Nachtrocknung bei einer Temperatur im Bereich von etwa 15 bis 50 °C in einer Gasatmosphäre oder im Vakuum unterzieht und/oder mit wenigstens einem Trocknungsmittel versetzt.

13. Verfahren nach einem der Ansprüche 11 und 12, **dadurch gekennzeichnet, dass** man als Trockengut ein Pulverkonzentrat mit einem Gehalt an lebensfähigen Mikroorganismen von etwa 4·10¹¹ bis 1.10¹² cfu/g erhält.

14. Trockene, verdichtete Mikroorganismenkultur nach einem der Ansprüche 1 bis 8, **dadurch gekennzeichnet, dass** sie **dadurch** erhältlich ist, dass man ein Pulverkonzentrat einer Mikroorganismenkultur gemäß einem der Ansprüche 9 und 10 verdichtet.

15. Verfahren zur Herstellung einer trockenen, verdichteten Mikroorganismenkultur gemäß einem der Ansprüche 1 bis 8, **dadurch gekennzeichnet, dass** man
i) eine flüssige Mikroorganismenkultur durch trägergebundene Sprühtrocknung gemäß einem der Ansprüche 11 bis 13 in ein Pulverkonzentrat der Mikroorganismenkultur überführt, welches 4·10¹¹ bis 1.10¹² cfu/g der Mikroorganismen enthält und das Trockengut eine Wasseraktivität a_{w} von weniger als 0,4 aufweist,und
ii) diese Mischung kompaktiert, tablettiert oder agglomeriert.

16. Verfahren nach Anspruch 15, **dadurch gekennzeichnet, dass** man das kompaktierte Pulverkonzentrat aus Stufe ii) bricht und gegebenenfalls klassiert.

17. Verwendung einer Mikroorganismenkultur oder eines Pulverkonzentrats einer Mikroorganismenkultur nach einem der Ansprüche 1 bis 10 als Starterkultur für Nahrungs- und Futtermittel.

## Claims

1. A dry microorganism culture which comprises at least one microorganism species in carrier-bound form, wherein the culture is present in the form of particles which
a) have a particle size of at least about 0.1 mm;
b) comprise from about 10¹⁰ to 4.10¹¹ cfu/g of at least one microorganism species; and
c) are compressed.

2. The microorganism culture according to claim 1, wherein the particles were compacted using a line lead of from about 5 to 15 kN/cm or tableted using a pressure of from about 90 to 160 mPa.

3. The microorganism culture according to claim 1 or 2 wherein it comprises compacted broken material in the form of compressed, comminuted particles having a diameter of from about 0.1 mm to about 2 mm.

4. The microorganism culture according to claim 1, wherein the compressed particles comprise tablets having a diameter of from about 2 to 50 mm and a ratio of diameter to thickness of from about 1:0.1 to about 10:1.

5. The microorganism culture according to one of the preceding claims, wherein it comprises, as further component, an effervescent additive.

6. The microorganism culture according to one of the preceding claims, wherein, as carrier, it comprises at least one matrix material for embedding the microorganism cells and, if appropriate, at least one further cell-stabilizing additive.

7. The microorganism culture according to one of the preceding claims, wherein it comprises at least one lactic-acid-producing bacterial species.

8. The microorganism culture according to claim 7, wherein the bacterial species is selected from bacteria of the genus Lactobacillus sp.

9. A powder concentrate of a microorganism culture which comprises from 4.10¹¹ to 1.10¹² cfu/g of at least one microorganism species and a carrier.

10. The powder concentrate according to claim 9, wherein it has a water activity a_{w} of less than 0.4.

11. A process for producing a powder concentrate according to claim 9 or 10, which comprises
a) dissolving or suspending at least one substance suitable for forming a carrier in a liquid comprising at least one microorganism species,
b) drying the resultant mixture in a spray dryer, for the spray-drying use being made of a conditioned dried gas having a dew point of from about -10 to about -50°C, which is heated to a temperature in the range of above about 80°C, and
c) removing the dried material from the spray dryer, this dried material having an exit temperature of from about 45 to 75°C.

12. The process according to claim 11, wherein, in a further stage d), the dry material is subjected to a further drying at a temperature in the range from about 15 to 50°C in a gas atmosphere or in vacuo and/or at least one desiccant is added.

13. The process according to either of claims 11 and 12, wherein, as dry material, a powder concentrate having a content of viable microorganisms of from about 4.10¹¹ to 1.10¹² cfu/g is obtained.

14. A dry compressed microorganism culture according to claims 1 to 8, wherein it is obtainable by compressing a powder concentrate of a microorganism culture according to one of claims 9 and 10.

15. A process for producing a dry compressed microorganism culture according to one of claims 1 to 8, which comprises
i) converting a liquid microorganism culture by carrier-bound spray drying according to one of claims 11 to 13 into a powder concentrate of the microorganism culture which comprises from 4.10¹¹ to 1.10¹² cfu/g of the microorganisms and the dry material has a water activity a_{w} of less than 0.4, and
ii) compacting, tableting or agglomerating this mixture.

16. The process according to claim 15, wherein the compacted powder concentrate from stage ii) is broken, with or without classification.

17. The use of a microorganism culture or of a powder concentrate of a microorganism culture according to one of claims 1 to 10 as starter culture for foodstuffs and feedstuffs.

## Revendications

1. Culture de micro organismes sèche, contenant au moins une espèce de micro organismes sous forme supportée, **caractérisée en ce que** la culture est présente sous la forme de particules,
a) qui présentent une taille de particule d'au moins environ 0,1 mm;
b) qui contiennent environ de 10¹⁰ à 4*10¹¹ cfu/g d'au moins une espèce de micro organisme ; et
c) qui sont condensés.

2. Culture de micro organismes selon la revendication 1, **caractérisée en ce que** les particules ont été compactées avec une force de ligne d'environ 5 à 15 kN/cm ou ont été mises sous la forme de comprimés à une pression d'environ 90 à 160 MPa.

3. Culture de micro organismes selon la revendication 1 ou 2, **caractérisée en ce qu'**elle comprend une matière broyée sous la forme de particules fragmentées, compactées ayant un diamètre d'environ 0,1 mm à d'environ 2 mm.

4. Culture de micro organismes selon la revendication 1, **caractérisée en ce que** les particules compactées comprennent des comprimés ayant une diamètre d'environ 2 à 50 mm et un rapport diamètre à épaisseur d'environ 1:0,1 à environ 10:1.

5. Culture de micro organismes selon l'une quelconque des revendications précédentes, **caractérisé en ce qu'**elle comprend, en tant que composant supplémentaire, un additif effervescent.

6. Culture de micro organismes selon l'une quelconque des revendications précédentes, **caractérisée en ce qu'**elle comprend, en tant que support au moins un matériau de matrice en vue du noyage des cellules de micro organismes et, le cas échéant, au moins un additif supplémentaire, stabilisant les cellules.

7. Culture de micro organismes selon l'une quelconque des revendications précédentes, **caractérisée en ce qu'**elle comprend au moins une espèce bactérienne produisant de l'acide lactique.

8. Culture de micro organismes selon la revendication 7, **caractérisée en ce que** l'espèce bactérienne est choisie parmi les bactéries du genre Lactobacillus sp.

9. Concentré pulvérulent d'une culture de micro organismes, contenant de 4*10¹¹ à 1*10¹² cfu/g d'au moins une espèce de micro organismes et d'un support.

10. Concentré pulvérulent selon la revendication 9, **caractérisé en ce qu'**il présente une activité d'eau a_{w} de moins de 0,4.

11. Procédé en vue de la fabrication d'un concentré pulvérulent selon la revendication 9 ou 10, **caractérisé en ce**
a) que l'on dissout ou l'on suspend, dans un liquide contenant au moins une espèce de micro organismes, au moins une substance appropriée à la formation d'un support,
b) que l'on sèche le mélange ainsi obtenu dans un sécheur par pulvérisation, moyennant quoi l'on utilise, en vue du séchage par pulvérisation, un gaz séché conditionné, ayant un point de rosée d'environ -10 à environ -50°C, qui a été chauffé à une température dans le domaine de plus d'environ 80°C, et
c) que l'on retire la matière sèche hors du sécheur par pulvérisation, moyennant quoi celle-ci présente une température de sortie d'environ 45 à 75°C.

12. Procédé selon la revendication 11, **caractérisé en ce que** l'on soumet, dans une étape supplémentaire d), la matière sèche à un post-séchage à une température dans le domaine d'environ 15 à 50°C, dans une atmosphère gazeuse ou sous vide, et/ou **en ce que** l'on la met en contact avec d'au moins un agent de séchage.

13. Procédé selon l'une quelconque des revendications 11 et 12, **caractérisé en ce que** l'on obtient, en tant que matière sèche, un concentré pulvérulent ayant une teneur en micro organismes vivants d'environ 4*10¹¹ à 1*10¹² cfu/g.

14. Culture de micro organismes séchée, compactée selon l'une quelconque des revendications 1 à 8, **caractérisée en ce qu'**elle est obtenue **en ce que** l'on compacte un concentré pulvérulent d'une culture de micro organismes conformément à l'une des revendications 9 et 10.

15. Procédé en vue de la fabrication d'une culture de micro organismes sèche, compactée selon l'une quelconque des revendications 1 à 8, **caractérisé**
i) en ce que l'on convertit une culture de micro organismes liquide par séchage par pulvérisation lié à un support selon l'une quelconque des revendications 11 à 13, pour former un concentré pulvérulent de la culture de micro organismes, qui présente de 4*10¹¹ à 1*10¹² cfu/g des micro organismes et en ce que la matière sèche présente une activité d'eau a_{w} de moins de 0,4, et
ii) en ce que ce mélange est compacté, mis sous forme de comprimés ou aggloméré.

16. Procédé selon la revendication 15, **caractérisé en ce que** l'on procède au broyage du concentré pulvérulent compacté provenant de l'étape ii) et, le cas échéant, à sa classification.

17. Utilisation d'une culture de micro organismes ou d'un concentré pulvérulent d'une culture de micro organismes selon l'une quelconque des revendications 1 à 10, en tant que culture de départ pour produits alimentaires et produits fourragers.
